Europäisches Patentamt

**European Patent Office**

Office européen des brevets

(19)

(11) Numéro de publication: **0 085 005**
**B1**

(12) **FASCICULE DE BREVET EUROPEEN**

(45) Date de publication du fascicule du brevet:
**08.01.86**

(21) Numéro de dépôt: **83400141.4**

(22) Date de dépôt: **20.01.83**

(51) Int. Cl.⁴: **C 07 K 3/22**, C 07 K 3/28,
C 07 K 15/06

(54) **Procédé de séparation d'immunoglobulines à partir du colostrum.**

(30) Priorité: **27.01.82 FR 8201230**

(43) Date de publication de la demande:
**03.08.83 Bulletin 83/31**

(45) Mention de la délivrance du brevet:
**08.01.86 Bulletin 86/2**

(84) Etats contractants désignés:
**AT BE CH DE FR GB IT LI LU NL SE**

(73) Titulaire: **FROMAGERIES BEL, 4, rue d'Anjou,
F-75008 Paris (FR)**

(72) Inventeur: **Goudal, Raymond Résidence du Parc Appt
A1-35, 12 Faubourg Chartrain, F-41100 Vendome (FR)**
Inventeur: **Huart, Philippe, 3 Rue des Frères Lumières
Appt 60, F-41100 Vendome (FR)**
Inventeur: **Sanchez, Victor, 1 Rue Léonard de Vinci,
F-31520 Ramonville-Saint-Agne (FR)**
Inventeur: **Mahenc, Jean, Labastide Falgarde La Croix
Falgarde, F-31120 Portet sur Faronne (FR)**

(74) Mandataire: **Polus, Camille et al, c/o Cabinet
Lavoix 2, Place d'Estienne d'Orves, F-75441 Paris
Cedex 09 (FR)**

(56) Documents cités:
FR - A - 2 095 728

CHEMICAL ABSTRACTS, vol. 79, no. 17, 29 octobre
1973, page 286, no. 103430a, Columbus, Ohio, US M.
JANUSZ et al.: "Immunoglobulins of colostrum. I.
Preparation and identification of ovine colostral
immunoglobulins"
CHEMICAL ABSTRACTS, vol. 79, no. 17, 29 octobre
1973, page 286, no. 103431b, Columbus, Ohio, US M.
JANUSZ et al.: "Immunoglobulins of colostrum. II.
Preparation and identification of bovine colostral
immunoglobulins"

(56) Documents cités: (suite)
CHEMICAL ABSTRACTS, vol. 85, no. 9, 30 août 1976,
page 426, no. 61211w, Columbus, Ohio, US S.S. Stone et
al.: "Chromatographic separation of gram quantities of
immunoglobulins from porcine colostrum against
transmissible gastroenteritis virus"
CHEMICAL ABSTRACTS, vol. 77, no. 17, 23 octobre
1972, page 285, no. 112325f, Columbus, Ohio, US C.
Sarmanioti et al.: "Chromatographic fractionation of
horse serum and colostral globulins and separation of
immunoglobulins G and G(T)"

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit
être formée par écrit et motivée. Elle n'est réputée formée qu'après paiement de la taxe d'opposition (Art. 99(1) Convention sur le
brevet européen).

## Description

La présente invention est relative à un procédé de séparation d'immunoglobulines à partir du colostrum.

Le colostrum est la sécrétion mammaire des mammifères femelles, dans les jours qui suivent la mise bas.

Sa composition et son aspect sont très différents de ceux du lait et ceci jusqu'au septième jour. Il se caractérise, entre autres, par sa richesse en protéines, notamment en immunoglobulines; le taux de ces dernières protéines varie de 6 à 10% à la première traite à 0,09%, qui est le taux contenu dans le lait. Or, ces protéines, et notamment les immunoglobulines, sont d'un grand intérêt, notamment dans le domaine médical et pharmaceutique. Elles peuvent, en particulier, être incorporées aux aliments du veau nouveau-né et servir à sa protection immunitaire, ou être utilisées en médecine, pharmacie ou alimentation humaine, notamment pour les produits de l'enfance. On a calculé que la quantité d'immunoglobulines contenues dans le colostrum produite au cours des sept traites consécutives équivalait à celle contenue dans le sang rejeté par les abattoirs.

Il est donc important de trouver un procédé permettant de séparer les immunglobulines présentes dans le colostrum.

Dans Chemical Abstracts V. 85, 1976, 61211w on a déjà décrit un procédé de séparation de faibles quantités d'immunoglobulines à partir d'un colostrum par chromatographie sur Sephadex® G.200 et filtration sur gel.

Dans la demande de brevet FR 2 321 932, et son certificat d'addition FR 2 359 634, on a proposé un procédé de séparation des protéines par chromatographie d'échange d'ions. On utilise, à cet effet, des échangeurs d'ions constitués par des particules poreuses à la surface desquelles sont greffés des groupements d'échangeurs d'ions.

En particulier, on a décrit dans la demande de brevet FR 3 321 932 la séparation d'immunoglobulines à partir de sérum humain délipidé sur un échangeur anionique (constitué par des particules de silice revêtues d'un polymère ayant des sites anioniques), les immunoglobulines étant récupérées dans l'effluent «à l'état électrophorétiquement pur», tandis que les autres protéines sont fixées.

Or, lorsqu'on cherche à opérer de la même façon avec un sérum de colostrum, on n'obtient dans l'effluent qu'une fraction enrichie en immunoglobulines, qui comprend encore une proportion importante d'autres protéines,

La Demanderesse a découvert qu'il était possible de séparer les immunoglobulines présentes dans le colostrum si, avant d'effectuer la chromatographie d'échange sur des échangeurs d'anions, on soumet un lait de colostrum ou un sérum de colostrum à un fractionnement par électrophorèse.

Ainsi, la présente invention a pour objet un procédé de séparation des immunoglobulines présentes dans le colostrum, caractérisé en ce que l'on soumet un lait de colostrum ou un sérum de colostrum à un fractionnement par électrophorèse, on récupère la fraction enrichie en immunoglobulines, puis on fait passer cette fraction enrichie en immunoglobulines directement, sans modifier le pH, à travers une colonne de particules échangeuses d'anions et on recueille les immunoglobulines dans l'effluent sortant de la colonne.

La matière première utilisée est, de préférence, un colostrum de vache, mais peut être tout colostrum issu de n'importe quel mammifère.

Le fractionnement ne s'effectue pas sur un colostrum entier, mais sur un lait de colostrum, auquel cas le colostrum original aura subi un écrémage ou, de préférence, sur un sérum de colostrum, auquel cas le colostrum aura subi un écrémage et une coagulation, afin d'éliminer la caséine. Cette coagulation peut être effectuée par adjonction de présure d'une force de 1/10000, à raison d'environ 0,1 ml/l ou par précipitation acide.

Le premier fractionnement utilisé dans la présente invention est un fractionnement par électrophorèse.

Ce type de fractionnement consiste, d'une manière générale, à fractionner une solution comprenant au moins deux groupes de substances dissoutes dans un liquide, en vue d'obtenir au moins deux fractions liquides contenant l'une le premier groupe de substances avec une richesse relative supérieure à celle de la solution initiale, l'autre le deuxième groupe avec une richesse relative supérieure à celle de ladite solution; pour cela, on délivre la solution initiale dans au moins une chambre de fractionnement délimitée sur deux grandes faces opposées par deux membranes semi-perméables, on applique un champ électrique dans ladite chambre, de façon à engendrer une migration des groupes de substances dépendant de leurs caractéristiques électriques et physiques provoquant par l'effet des gradients de concentration une convection naturelle avec la création de flux liquides ascendants et descendants, et on soutire le liquide en au moins deux zones de prélèvement différentes de la chambre en vue de recueillir lesdites fractions.

Ce fractionnement peut, en particulier, être réalisé selon le procédé décrit dans le brevet FR 80/23 952 et la thèse de Docteur-Ingénieur de la Faculté des Sciences de Toulouse: Préparation de fractions protéiques du sérum ou du plasma sanguin par électrophorèse, P. Espenan, 1980. Dans ce procédé, on utilise un champ électrique pour réaliser à travers au moins une des membranes semi-perméables un échange ionique à vitesse élevée entre la solution initiale contenue dans la ou les chambres de fractionnement et une solution auxiliaire disposée de l'autre côté de ladite membrane et contenant des ions de nature appropriée pour passer à travers ladite membrane et accentuer les différences de migrations des substances de la solution initiale.

Selon un mode de mise en œuvre préférentiel de ce procédé, la solution initiale est admise dans une série de chambres de fractionnement accolées séparées les unes aux autres par les membranes semi-perméables, la solution auxiliaire étant disposée de part et d'autre des deux chambres extrêmes dans deux compartiments de garde, séparés chacun par une membrane semi-perméable de la chambre de fractionnement extrême voisine, les échanges ioniques s'effectuant à vitesse élevée sous l'effet du champ électrique, à partir desdits compartiments de garde, de proche en proche dans les diverses chambres de fractionnement.

Ainsi, ce procédé conduit à réaliser en une seule opération, dans le même dispositif, d'une part, une adaptation de la solution initiale pour lui conférer des propriétés appropriées de nature à accentuer les différences de migration des substances, d'autre part, l'opération de fractionnement proprement dit desdites substances. Le champ électrique servant au fractionnement sert également à engendrer des échanges ioniques rapides à travers les membranes entre la solution initiale contenue dans les diverses chambres de fractionnement et la solution auxiliaire contenue dans les compartiments de garde. On supprime ainsi radicalement les opérations de préparation préalables, notamment de dialyse qui sont nécessaires dans les procédés classiques, ainsi que tous les inconvénients afférents; de plus, la solution initiale n'a plus à être diluée et le procédé peut s'appliquer à des solutions naturelles dans l'état où elles sont produites ou recueillies.

Ce procédé à déjà été utilisé pour le fractionnement et la récupération des immunoglobulines du plasma en vue de séparer une fraction riche en albumine et une fraction riche en γ-globulines.

Dans la présente invention, la séparation par électrophorèse est effectuée avantageusement à un pH de 6 à 7, de préférence à pH 6,3-6,4 (c'est-à-dire en pratique généralement sans modification du pH) et à une température de 4°C à 25°C, de préférence à 4°C. On récupère, à la partie supérieure du dispositif d'électrophorèse, une fraction relativement plus riche en immunoglobulines à l'état natif que la concentration initiale et, à sa partie inférieure, une fraction relativement plus riche en albumines. A titre d'exemple, si on a une solution ayant la composition suivante (en % en poids):

|  |  |  |
|---|---|---|
| - teneur en immunoglobulines | | 77% |
| - | α-lactalbumine | 4,5% |
| - | β-lactoglobuline | 14,4% |
| - | sérum-albumines | 3,0% |

on obtient, respectivement, la composition suivante (en % en poids) dans les fractions relativement plus riches en immunoglobulines et en albumines.

|  | Fraction riche en immuno-globulines (haut du dispositif) | Fraction riche en albumines (bas du dispositif) |
|---|---|---|
| Immunoglobulines | 83 | 67 |
| α-lactalbumine | 1,2 | 6,4 |
| β-lactoglobuline | 8,0 | 20,0 |
| Sérumalbumines | — | 5,6 |

La quantité récupérée d'immunoglobulines en haut du dispositif dépend des conditions opératoires et, notamment, du débit de soutirage; elle peut atteindre 70 à 80%.

Le second fractionnement utilisé dans la présente invention est un fractionnement par chromatographie d'échange sur des échangeurs d'anions. Il consiste à soumettre à une chromatographie d'échange sur des échangeurs d'anions la fraction en immunoglobulines obtenue par le premier fractionnement.

A cet effet, on fait percoler sur une colonne remplie de particules échangeuses d'ions la fraction riche en immunoglobulines.

Comme particules échangeuses d'anions, on utilise avantageusement des particules poreuses vendues sous le nom de «Sphérosil® , Q MA, échangeur d'ions basique fort», par la Société RHONE-POULENC et décrites dans les brevets FR 2 321 932 et FR 2 359 634.

Les Sphérosil Q MA, sont obtenus par fixation de résines échangeuses d'anions sur des billes de silice poreuses (X OB 0 15).

Toute autre type d'échangeur d'anions permettant la fixation sélective des protéines peut être utilisé, par exemple des celluloses greffées, telles que les résines Trisacryl échangeuses d'anions vendues par I B F.

Les échangeurs d'anions, tels que des échangeurs Sphérosil® Q MA permettent de traiter directement la fraction riche en immunoglobulines obtenue au premier stade, sans modification du pH (entre 6 et 7), et d'obtenir, avec un rendement élevé, les immunoglobulines dans l'effluent sortant de la colonne. Ceci présente l'avantage d'éviter tout risque de dénaturation des immunoglobulines par fixation sur les échangeurs d'ions.

On peut ainsi obtenir une solution de protéines comprenant de 98 à 100% d'immunoglobulines.

La chromatographie peut être effectuée à une température de 4 à 25°C et, avantageusement, à 4°C.

La solution d'immunoglobulines peut ensuite être post-traitée, par séchage, concentration, dialyse ou ultrafiltration, par exemple en vue de l'utilisation ultérieure.

La présente invention est illustrée par les exemples suivants.

*Exemple 1*

A - *Fractionnement par électrophorèse*

On part d'un sérum de colostrum ayant un pH de 6,3 et ayant la composition suivante (en g/l)

|  |  |
|---|---|
| - Immunoglobulines | 65,5 |
| - α-lactalbumine | 8,7 |
| - β-lactoglobuline | 15,2 |
| - Sérumalbumines | 6,3 |

On soumet ce sérum de colostrum à un fractionnement par électrophorèse en utilisant le dispositif décrit dans le brevet FR-A-2 493 725. On opère à une température de 4°C.

Ce dispositif comprend cinq chambres de fractionnement séparées par des membranes semi-perméables en cellophane. Chaque membrane a une surface active de 60 cm². Chaque chambre à une épaisseur de 3 mm et comprend un cadre délimitant onze canaux verticaux contre lesquels s'appuient les membranes.

La solution auxiliaire circulant dans les compartiments de garde latéraux est un mélange de $Na_2HPO_4$ et $KH_2PO_4$ à une concentration saline de 0,5 M et à un pH de 6,25. Dans les compartiments d'électrodes adjacents circule également une solution de $N_2HPO_4$ et $KH_2PO_4$, mais à une concentration de 0,2 M.

On a fait trois essais dont les conditions opératoires sont données dans le tableau I.

TABLEAU I

|  | Débit de soutirage sortie haut (ml/h) | Débit de soutirage sortie bas (ml/h) | Différentiel de potentiel entre les 2 membranes extrêmes des chambres de soutirage | Intesité du courant |
|---|---|---|---|---|
| Essai I | 105 | 105 | 6 V | 0,8 A |
| Essai II | 70 | 70 | 6 V | 0,8 A |
| Essai III | 105 | 35 | 6 V | 0,77 A |

Pour chaque essai, deux litres de sérum de colostrum sont traités par électrophorèse.

Dans le tableau II, on donne la quantité de chaque protéine récupérée en haut et en bas du dispositif, ainsi que le volume de la solution recueillie.

TABLEAU II

|  | Essai I | | Essai II | | Essai III | |
|---|---|---|---|---|---|---|
|  | Haut | Bas | Haut | Bas | Haut | Bas |
| Immunoglobulines | 68 | 63 | 75 | 52 | 102 | 27 |
| α-lactalbumine | 6 | 11,4 | 4,8 | 12,2 | 7 | 10 |
| β-lactaglobuline | 6 | 24,4 | 5,3 | 25,0 | 7 | 22,2 |
| Sérumalbumines | 0,6 | 11,9 | 0,5 | 11,8 | 1 | 11,2 |
| Volume recueilli (en litres) | 1 | 1 | 1 | 1 | 1,5 | 0,5 |

On constate que la fraction recueillie en haut du dispositif est plus concentrée en immunoglobulines que la fraction initiale.

La quantité d'immunoglobulines récupérées en haut du dispositif dépend des conditions expérimentales. Dans le troisième essai, elle approche 80%.

Une analyse d'immunodiffusion radiale (méthode Mancini) montre que la dénaturation est très faible. Par immunoélectrophorèse, on constate que l'on recueille toutes les immunoglobulines présentes dans le colostrum d'alimentation (IgG$_1$, IgA et IgM).

B - *Fractionnement par chromatographie d'echange d'ions*

On fait percoler 200 ml de liquide obtenu dans l'essai I à la sortie «haut» du dispositif et enrichi en immunoglobulines sur une colonne de Sphérosil Q MA échangeuse d'anions, contenant 64 g de Sphérosil Q MA à pH 6,3 et à une température de Sphérosil de 4°C. Le débit est de 300 ml/h.

Les résultats obtenus sont donnés dans le tableau III.

TABLEAU III

|  | Alimentation colonne Grammes de protéines | Effluent grammes de protéines | Fixation sur colonne Grammes de protéines |
|---|---|---|---|
| Immunoglobulines | 13,6 | 8 à 10 | 3,6 à 5,6 |
| α-lactalbumine | 1,2 | 0 | 1,2 |
| β-lactoglobulines | 1,2 | 0 | 1,2 |
| Sérumalbumines | 0,12 | 0 | 0,12 |

L'effluent ne contient que des immunoglobulines. De plus, l'analyse d'électroimmunodiffusion indique que ce sont exclusivement des IgG$_1$ et, plus spécifiquement, la fraction des IgG$_1$ dont le point isoélectrique est le plus élevé. Cette fraction devrait également contenir les lactotransferrines dont le point isoélectrique est de 7,8.

Par élution avec HCl 0,1 N, on recupère les albumines, ainsi que les autres immunoglobulines IgG$_1$, IgA et IgM.

Des résultats analogues sont obtenus avec le liquide sortant en haut de la cellule d'électrophorèse dans les essais II et III.

La capacité de fixation moyenne des sphérosils Q MA est, dans le cas présente, de 110 mg/g.

*EXEMPLE COMPARATIF*

On part d'un échantillon de 250 ml de sérum de colostrum (provenant de la première traite), qui a une teneur totale en protéines de 104 g/l et la composition suivante en immunoglobulines et en sérum-albumines.

| | en % | soit en grammes de protéines traitées |
|---|---|---|
| Immunoglobulines | 77,7 | 19,4 |
| α-lactalbumine | 2,4 | 0,6 |
| β-lactaglobuline | 15,4 | 3,8 |
| Sérum-albumines | 3,0 | 0,8 |

On fait percoler directement ce sérum de colostrum sur une colonne de Sphérosil® Q MA échangeuse d'anions contenant 64 g de résine (identique à celle utilisée à l'exemple 1). La capacité de fixation est de 96 mg/g à pH 6,3 à 4°C et le débit de 300 ml/h.

L'effluent et l'éluat obtenu avec une solution HCl 0,1 N ont la composition suivante:

| | Effluent | | Eluat | |
|---|---|---|---|---|
| | % | Grammes récupérés | % | Grammes récupérés |
| Immunoglobulines | 89,6 | 15,4 | 45,4 | 2,5 |
| α-lactalbumine | 1,5 | | | |
| β-lactaglobuline | 8,2 | 1,6 | 54,6 | 3,0 |
| Sérumalbumines | 0,7 | | | |

On constate que l'effluent s'est enrichi en immunoglobulines, mais encore une fraction importante d'autres protéines.

**Revendications**

1. Procédé de séparation des immunoglobulines présentes dans le colostrum, caractérisé en ce que l'on soumet un lait de colostrum ou un sérum de colostrum à un fractionnement par électrophorèse, on récupère la fraction enrichie en immunoglobulines, puis on fait passer cette fraction enrichie en immunoglobulines directement, sans modifier le pH, à travers une colonne de particules échangeuses d'anions et on recueille les immunoglobulines dans l'effluent sortant de la colonne.

2. Procédé selon la revendication 1, caractérisé en ce que la matière traitée est un sérum de colostrum.

3. Procédé selon la revendication 1 ou 2, caractérisé en ce que l'on effectue le fractionnement par électrophorèse à un pH de 6 à 7.

4. Procédé selon l'une quelconque des revendications précédentes, caractérisé en ce que l'on effectue le fractionnement par électrophorèse à un pH de 6,3-6,4.

5. Procédé selon l'une quelconque des revendications précédentes, caractérisé en ce que l'on effectue le fractionnement par électrophorèse à une température de 4 à 25°C.

6. Procédé selon l'une quelconque des revendications précédentes, caractérisé en ce que l'on effectue le fractionnement par chromatographie d'échange d'ions à une température de 4 à 25°C.

**Patentansprüche**

1. Verfahren zur Abtrennung von in dem Kolostrum vorhandenen Immunoglobulinen, dadurch gekennzeichnet, dass man eine Kolostrum-Milch oder ein Serum des Kolostrums einer Fraktionierung mittels Elektrophorese unterwirft, man die mit den Immunoglobulinen angereicherte Fraktion wiedergewinnt, man anschliessend diese mit Immunoglobulinen angereicherte Fraktion direkt, ohne den pH-Wert zu verändern, durch eine Kolonne mit Anionenaustauscherteilchen passieren lässt und man die Immunoglobine in dem aus der Kolonne ausströmenden Medium sammelt.

2. Verfahren nach Anspruch 1, dadurch gekennzeichnet, dass die behandelte Substanz ein Serum des Kolostrums ist.

3. Verfahren nach einem der Ansprüche 1 oder 2, dadurch gekennzeichnet, dass man die Fraktionierung durch Elektrophorese bei einem pH-Wert von 6 bis 7 durchführt.

4. Verfahren nach einem der vorhergehenden Ansprüche, dadurch gekennzeichnet, dass man die Fraktionierung durch Elektrophorese bei einem pH-Wert von 6,3 bis 6,4 durchführt.

5. Verfahren nach einem der vorhergehenden Ansprüche, dadurch gekennzeichnet, dass man die Fraktionierung durch Elektrophorese bei einer Temperatur von 4 bis 25°C durchführt.

6. Verfahren nach einem der vorhergehenden Ansprüche, dadurch gekennzeichnet, dass man die Fraktionierung durch Ionenaustauschchromatographie bei einer Temperatur von 4 bis 25°C durchführt.

**Claims**

1. A process for the separation of the immunoglobulins present in colostrum, characterised in that a colustrum milk or a colustrum serum is fractionated by electrophoresis, the immunglobulin-enriched fraction is recovered, then this immunoglobulin-enriched fraction is percolated directly, without modifying the pH, through a column of anion exchanger particles and the immunoglobulins are collected in the effluent that leaves the column.

2. A process as claimed in claim 1, characterised in that the substance treated is a colustrum serum.

3. A process as claimed in claim 1, characterised in that elctrophoresis fractionation is performed at a pH of 6 to 7.

4. A process as claimed in claim 1, characterised in that electrophoresis fractionation is performed at a pH of 6.3 - 6.4.

5. A process as claimed in claim 1, characterised in that electrophoresis fractionation is performed at a temperature of 4 to 25°C.

6. A process as claimed in any one of the preceding claims, characterised in that ion-exchange chromatography fractionation is performed at a temperature of 4 to 25°C.